# EUROPÄISCHE PATENTANMELDUNG

(11) **EP 3 666 375 A1**
(43) Veröffentlichungstag der Anmeldung: **17.06.2020**
(21) Anmeldenummer: 18212331.5
(22) Anmeldetag: 13.12.2018
(51) Int. Cl.: B01J 8/04, C07C 1/12, B01J 35/00, B01J 23/46, B01J 23/89

(54) **ROHRREAKTOR UND VERFAHREN ZUM BETREIBEN EINES ROHRREAKTORS**

(71) Anmelder: Siemens Aktiengesellschaft, 80333 München (DE); Friedrich-Alexander-Universität Erlangen-Nürnberg, 91054 Erlangen (DE)
(72) Erfinder: Albert, Jakob, 91054 Erlangen (DE); Baldauf, Manfred, 91056 Erlangen (DE); Meltzer, Katharina, 91052 Erlangen (DE); Stiegler, Thomas, 91054 Erlangen (DE); Tremel, Alexander, 91096 Möhrendorf (DE); Wasserscheid, Peter, 91054 Erlangen (DE)

(57) **Zusammenfassung**

Die Erfindung betrifft ein Verfahren zum Betreiben eines Rohrreaktors und einen Rohrreaktor zum Umsetzen von Wasserstoff zu Methan umfassend eine erste Reaktionszone mit einer metallischen Trägerstruktur, die mit einem Ruthenium umfassenden Material beschichtet ist, mit einer Temperatur in einem ersten Temperaturbereich, eine zweite Reaktionszone mit einer metallischen Trägerstruktur, die mit einer Ruthenium umfassenden Material ausgestaltet ist, oder mit einem als Schüttung ausgeführten Ruthenium umfassenden Katalysatorbett mit einer Temperatur in einem zweiten Temperaturbereich, und eine dritte Reaktionszone mit einem als Schüttung ausgeführten Katalysatorbett umfassend Nickel als Katalysator mit einer Temperatur in einem dritten Temperaturbereich.

## Beschreibung

Die Erfindung betrifft einen Rohrreaktor zum Umsetzen von Wasserstoff zu Methan und ein Verfahren zum Betreiben des Rohrreaktors.

Die Stromerzeugung schwankt mit zunehmendem Anteil an Strom aus erneuerbaren Energien während des Tagesverlaufs. Um ein Überangebot an Strom in Zeiten mit viel Sonne und starkem Wind bei niedriger Nachfrage nach Strom ausgleichen zu können, benötigt man regelbare Kraftwerke oder Speicher, um diese Energie zu speichern.

Eine der derzeit angedachten Lösungen ist das Umwandeln von elektrischer Energie in Wertprodukte, die insbesondere organische Plattformchemikalien oder Synthesegas, welches Kohlenstoffmonoxid und Wasserstoff umfasst, darstellen. Eine mögliche Technik zur Umwandlung der elektrischen Energie in Wertprodukte stellt die Elektrolyse dar.

Die Elektrolyse von Wasser zu Wasserstoff und Sauerstoff stellt eine im Stand der Technik bekannte Methode dar. Der in dem Elektrolyseprozess hergestellte Wasserstoff kann mit einer Kohlenstoffdioxid umfassenden Komponente zu Methan und Wasser reagieren. Das Methan kann dann vorteilhaft direkt in die bestehende Erdgasinfrastruktur eingespeist werden.

Da die regenerativen Energiequellen wie Windenergie und Solarenergie nicht regelmäßig bereitstehen, kommt es bei der Produktion von Wasserstoff aus der Elektrolyse zu Schwankungen hinsichtlich der produzierten Wasserstoffmenge. Diese Schwankungen führen zu Fluktuationen des Wasserstoffvolumenstroms in einem Methanisierungsreaktor und können daher bei der Methanisierungs-Reaktion nachteilig zu einer Fluktuation der Produktgasqualität führen. Dadurch ist es nachteilig nötig, das produzierte Erdgas vor einer Einspeisung in das Erdgasnetz aufzubereiten. Ein zusätzlicher Aufbereitungsprozess führt nachteilig zu einem komplexeren Gesamtaufbau der Anlage und zu höheren Prozesskosten.

Eine Möglichkeit diesen schwankenden Wasserstoffstrom auszugleichen besteht darin, einen Wasserstoffspeicher zwischen dem Wasserelektrolyseur und dem Reaktor zur Methanisierung anzuordnen. Die Wasserstoff-Fluktuationen werden auf diese Weise gepuffert. Nachteilig sind diese Wasserstoffspeicher sehr groß, wartungs- und kostenintensiv.

Trotz Einsatz eines Wasserstoffspeichers kann es dennoch zu langfristigen Unterbrechungen der Wasserstoffzufuhr kommen, sodass der Reaktor nicht betrieben werden kann. Um das Anfahren des Reaktors nach einer Unterbrechung möglichst kurzfristig gewährleisten zu können, werden die Reaktoren während der Stillstandzeit beheizt. Dieses Beheizen ist nachteilig kosten- und energieintensiv.

Aus der Literatur sind Rohrreaktoren bekannt, welche mehrere Zonen aufweisen. Die Zonen werden bei unterschiedlichen Temperaturebereichen betrieben, um einen dynamischen Betrieb bei schwankendem Wasserstoffstrom zu gewährleisten. Bisher werden insbesondere Nickel basierte Katalysatoren eingesetzt. Diese Katalysatoren nehmen allerdings, wenn sie eine ausreichende Aktivität erreichen sollen, große Volumina ein. Durch den erhöhten Platzbedarf werden die Reaktoren nachteilig groß und demzufolge auch kostenintensiv.

Aufgabe der vorliegenden Erfindung ist es daher, einen Reaktor und ein Verfahren zum Betreiben eines Reaktors zum Herstellen von Methan aus Wassererstoff anzugeben, welches eine kompakte, Bauweise aufweist und ein effizientes Verfahren ermöglicht.

Die Aufgabe wird mit einem Rohrreaktor gemäß Anspruch 1 und einem Verfahren zum Betreiben des Rohrreaktors gemäß Anspruch 13 gelöst.

Der erfindungsgemäße Rohrreaktor zum Umsetzen von Wasserstoff zu Methan umfasst eine erste Reaktionszone mit einer metallischen Trägerstruktur, die mit einem Ruthenium umfassenden Material beschichtet ist. In der ersten Reaktionszone herrscht eine Temperatur in einem ersten Temperaturbereich. Der Rohrreaktor umfasst weiterhin eine zweite Reaktionszone mit einer metallischen Trägerstruktur, die mit einem Ruthenium umfassenden Material beschichtet ist oder die zweite Reaktionszone umfasst ein als Schüttung ausgeführtes Katalysatorbett mit einem Ruthenium umfassenden Material. In der zweiten Reaktionszone herrscht eine Temperatur in einem zweiten Temperaturbereich. Der Rohrreaktor umfasst weiterhin eine dritte Reaktionszone mit einer als Schüttung ausgeführten Katalysatorbett umfassend Nickel als Katalysator. In der dritten Reaktionszone herrscht eine Temperatur in einem dritten Temperaturbereich.

Das erfindungsgemäße Verfahren umfasst mehrere Schritte. Zunächst wird ein Rohrrektor bereitgestellt. Der Rohrreaktor umfasst drei Reaktionszonen. Eine erste Reaktionszone mit einer metallischen Trägerstruktur, die mit einem Ruthenium umfassenden Material beschichtet ist. In der ersten Reaktionszone herrscht eine Temperatur in einem ersten Temperaturbereich. Der Rohrreaktor umfasst weiterhin eine zweite Reaktionszone mit einer metallischen Trägerstruktur, die mit einem Ruthenium umfassenden Material beschichtet ist oder die zweite Reaktionszone umfasst ein als Schüttung ausgeführtes Katalysatorbett umfassend Ruthenium. In der zweiten Reaktionszone herrscht eine Temperatur in einem zweiten Temperaturbereich. Der Rohrreaktor umfasst weiterhin eine dritte Reaktionszone mit einem als Schüttung ausgeführten Katalysatorbett umfassend Nickel als Katalysator. In der dritten Reaktionszone herrscht eine Temperatur in einem dritten Temperaturbereich. Ein Eduktgas umfassend Wasserstoff wird in die erste Reaktionszone geführt. In der ersten Reaktionszone wird ein erster Anteil des Wasserstoffs zu einem ersten Anteil Methan an der Ruthenium umfassenden Schicht umgesetzt. Der nicht reagierte Wasserstoff und der erste Anteil Methan werden in die zweite Reaktionszone geführt. In der zweiten Reaktionszone werden ein zweiter Anteil des Wasserstoffs zu einem zweiten Anteil Methan umgesetzt. Dabei ist der zweite Anteil Methan größer als der erste Anteil Methan. Der nicht reagierte Wasserstoff, der erste und der zweite Anteil an Methan werden aus der zweiten in die dritte Reaktionszone geführt. In der dritten Reaktionszone wird ein dritter Anteil Wasserstoff zu einem dritten Anteil Methan umgesetzt.

Vorteilhaft ermöglicht das Führen des Eduktgases umfassend Wasserstoff aufgrund der unterschiedlichen Reaktionszonen mit den erfindungsgemäß angeordneten Katalysatoren ein flexibles Umsetzen von Wasserstoff zu Methan und vermeidet dabei den Einsatz eines großen kostenintensiven Wasserstoffspeichers oder den Einsatz einer Temperierung während einer Reaktionsunterbrechung, sodass das flexible Umsetzen des Wasserstoffs bei geringem Energieverbrauch möglich ist.

Der erfindungsgemäße Rohrreaktor und das erfindungsgemäße Verfahren zum Betreiben eines Rohrreaktors ermöglichen weiterhin vorteilhaft eine dynamische Herstellung von Methan bei einer möglichst kompakten Bauweise des Rohrreaktors. Das Aufbringen des Rutheniums auf die metallische Trägerstruktur als Schicht ermöglicht vorteilhaft, dass sowohl für hohe Temperaturen als auch für variierende Wasserstoff-Volumenströme der erste Anteil an Wasserstoff und der zweite Anteil Wasserstoff derart kontinuierlich umgesetzt werden können, dass ein Abschalten des Rohrreaktors bei geringen Mengen von Wasserstoff vermieden werden kann.

Das Ruthenium weist eine höhere Aktivität im Vergleich zum Nickel für diese Reaktion auf. Die höhere Aktivität des Rutheniums im Vergleich zum Nickel für diese Reaktion ermöglichen kompakte Bauformen der ersten und zweiten Reaktionszone. Der dritte Anteil an Wasserstoff, welcher in der dritten und letzten Zonen umgesetzt wird, ist derart gering, dass dort Nickel in Form eines Katalysatorbetts vorteilhaft kostengünstig eingesetzt werden kann. Des Weiteren muss diese dritte Reaktionszone nicht derart dynamisch betrieben werden können wie die ersten und zweite Reaktionszone, da der Anteil an zur Verfügung stehenden Wasserstoff zu Beginn dieser Zone ein nahezu konstantes Niveau aufweist. Somit ist der erfindungsgemäße Rohrreaktor vorteilhaft sowohl von kompakter Bauweise, dynamisch betreibbar und möglichst kostengünstig mit Katalysator ausgestattet.

In einer vorteilhaften Ausgestaltung und Weiterbildung der Erfindung umfasst das Trägermaterial des Ruthenium-basierten Katalysators Al₂O₃, Al₂O₃/MgO, CeO₂ oder SiO₂. Vorteilhaft zeigen diese Trägermaterialien basisches Verhalten und sind somit für eine Methanisierung gut geeignet.

In einer vorteilhaften Ausgestaltung und Weiterbildung der Erfindung ist die metallische Trägerstruktur wabenförmig, porös oder als Diamanteinheitszelle ausgestaltet. Vorteilhaft ist das Verhältnis von beschichtbarer Oberfläche zu Gesamtvolumen möglichst groß. Die Poren, oder in anderen Worten Löcher, oder in anderen Worten Aussparungen, in der Trägerstruktur liegen typischerweise in einem Größenbereich von 3 nm bis 10 nm. Die Form der Aussparungen kann grundsätzlich die eines Vielecks sein. Besonders vorteilhaft kann die Trägerstruktur, insbesondere die Diamanteinheitszelle, mittels 3D-Druck (engl.: "Additive Manufacturing") hergestellt werden. Das Wählen einer günstigen Trägerstruktur gewährleistet vorteilhaft eine kompakte Bauweise der ersten und zweiten Reaktionszone.

In einer weiteren vorteilhaften Ausgestaltung und Weiterbildung der Erfindung sind in Strömungsrichtung des Wasserstoffs eingangsseitig die erste Reaktionszone, danach die zweite Reaktionszone und danach die dritte Reaktionszone angeordnet. Vorteilhaft ermöglicht der Einsatz des Rutheniums als Katalysator in der ersten und zweiten Reaktionszone zu Beginn des Rohrreaktors das dynamische Umsetzen des Wasserstoffs zu Methan ohne ein Abschalten des Reaktors aufgrund von geringer Eduktmenge oder aufgrund von Überhitzen des Reaktors durchführen zu müssen. Die dritte und letzte Reaktionszone wird mit günstigem Nickel im Katalysatorbett gefüllt, da eine dynamische Betriebsweise hier nicht mehr gefordert ist. Somit wird vorteilhaft ein möglichst kostengünstiger und kompakter Rohrreaktor eingesetzt.

In einer vorteilhaften Ausgestaltung und Weiterbildung der Erfindung liegt der zweite Temperaturbereich oberhalb des ersten Temperaturbereichs.

Die erste Reaktionszone stellt eine Anfahrzone dar. Der erste Temperaturbereich dieser Zone sollte zweckmäßigerweise über einer Mindesttemperatur liegen, die oberhalb der Reaktionszündtemperatur und oberhalb der Kondensationstemperatur von Wasser, insbesondere 100 °C, liegt.

Die zweite Reaktionszone weist eine Temperatur in einem zweiten Temperaturbereich auf. Diese sollte insbesondere unterhalb der Temperatur liegen, die die Stabilität des Katalysators begrenzt. In dieser Zone erfolgt der größte Umsatz des Wasserstoffs.

In der dritten Reaktionszone wird der Wasserstoff umgesetzt, welcher in den ersten beiden Reaktionszone noch nicht umgesetzt wurde. Vorteilhaft ist, wenn die Temperatur in dem dritten Temperaturbereich geringer als in dem zweiten Temperaturbereich ist. Wie bereits in der ersten Reaktionszone sollte die Temperatur in dieser dritten Reaktionszone zweckmäßigerweise aber oberhalb der Kondensationstemperatur von Wasser und oberhalb der Reaktionsstarttemperatur liegen.

In einer weiteren vorteilhaften Ausgestaltung und Weiterbildung der Erfindung liegt die Temperatur in der ersten Reaktionszone in einem ersten Temperaturbereich, der von 100 °C bis 400 °C reicht. Besonders vorteilhaft liegt die Temperatur in dem ersten Temperaturbereich zwischen 150 °C und 250 °C. Vorteilhaft wird die in der ersten Reaktionszone entstehende Wärme in die zweite Reaktionszone mittels der Edukte und Produkte transportiert und somit zum Heizen der zweiten Reaktionszone verwendet. Vorteilhaft kann in Abhängigkeit des Volumenstroms des Eduktgases die Eintrittstemperatur in die erste Reaktionszone, in die zweite Reaktionszone und/oder in die dritte Reaktionszone angepasst werden. Bei geringen Volumenströmen wird die Heizleistung in der ersten Zone niedriger eingestellt, da die Verweilzeit des Eduktgases in der ersten Reaktionszone höher ist und damit anteilig am Eduktgas mehr umgesetzt werden kann. Bei hohen Volumenströmen des Eduktgases durch die erste Reaktionszone wird die Eintrittstemperatur, insbesondere in die erste Reaktionszone höher eingestellt, da die Verweilzeit des Eduktgases in der ersten Reaktionszone kürzer ist. Weiterhin ist der anteilige, in anderen Worten relativ zur Eduktgasmenge, Umsatz geringer als bei niedrigen Eduktgasströmen.

In einer weiteren vorteilhaften Ausgestaltung und Weiterbildung der Erfindung beträgt die maximale Temperatur des zweiten Temperaturbereichs 600 °C. Besonders vorteilhaft beträgt die Maximaltemperatur des zweiten Temperaturbereichs 500 °C. Vorteilhaft wird diese Maximaltemperatur derart gewählt, dass sowohl die Festigkeit des Reaktormaterials als auch die Stabilität des Katalysators gewährleistet werden können. In dieser zweiten Reaktionszone wird ein hoher Umsatz von Wasserstoff mit Kohlenstoffdioxid zu Methan erzielt, was zu hohen Wärmemengen führt. Diese Wärme muss abgeführt werden, um die maximale Temperatur nicht zu überschreiten. Das kann mittels einer indirekten Kühlung, insbesondere durch das Einbringen von Kühlmedien durchströmten Bauteilen, insbesondere in Form von Wärmetauschern, erfolgen. Alternativ kann auch ein direktes Kühlen mittels eines Kühlfluids, insbesondere Wasser, durchgeführt werden, wobei das Wasser insbesondere in die zweite Reaktionszone eingedüst wird.

In einer weiteren vorteilhaften Ausgestaltung und Weiterbildung der Erfindung liegt die Temperatur im dritten Temperaturbereich zwischen 200 °C und 400 °C. Besonders vorteilhaft liegt die Temperatur im dritten Temperaturbereich zwischen 250 °C und 350 °C. Vorteilhaft liegt die Temperatur im dritten Temperaturbereich unterhalb der Temperatur des zweiten Temperaturbereichs bei hoher Katalysatorbettdichte, wodurch sichergestellt wird, dass der noch nicht umgesetzte Wasserstoff und das Kohlenstoffdioxid in der dritten Reaktionszone zu Methan umgesetzt wird.

In einer weiteren vorteilhaften Ausgestaltung und Weiterbildung der Erfindung umfasst die erste Reaktionszone eine Heizung. Diese Heizung gewährleistet vorteilhaft, dass die Minimaltemperatur für den Reaktionsstart erreicht wird. Weiterhin gewährleistet die Heizung, dass die Temperatur oberhalb der Kondensationstemperatur des Wassers liegt. Wasser wird als Produkt während des Umsetzens des Wasserstoffs mit Kohlenstoffdioxid zu Methan produziert. Zusätzliches Wasser kann nach Bedarf als Kühlfluid in den Rohrreaktor geführt werden. Das Wasser sollte dampfförmig vorliegen, um die Stabilität des Katalysatorbetts zu erhalten.

Die Metallstruktur ist vorteilhaft elektrisch leitend. Vorteilhaft weisen solche metallischen Grundstrukturen eine hohe Wärmeleitfähigkeit und eine hohe elektrische Leitfähigkeit auf. Weiterhin haben diese Strukturen ein hohes Oberflächenzu-Volumenverhältnis, was einen effektiven Wärmeeintrag in den Reaktor ermöglicht. Der Wärmeeintrag kann über eine Beheizung der Reaktorwand, welche mit der Metallstruktur in direktem Kontakt steht, erfolgen. Dies ermöglicht einen im Vergleich zu herkömmlichen Katalysatorpellets besseren Wärmeeintrag. Dies ist insbesondere in der ersten Reaktionszone vorteilhaft.

Alternativ kann die Metallstruktur mit Elektroden verbunden sein. Durch Anlegen einer Spannung an die Elektroden und den elektrischen Widerstand in der Metallstruktur kann ein Temperaturanstieg der Metallstruktur erreicht werden. Das Kontaktieren der Metallstruktur mit Elektroden ermöglicht einen noch schnelleren Wärmeeintrag im Vergleich zur Kontaktierung der Metallstruktur mit einer Heizung. Das schnelle Beheizen der ersten Reaktionszone ist besonders dann vorteilhaft, wenn der Reaktor bei einer Unterbrechung der Eduktzufuhr unterhalb einer Minimaltemperatur abgekühlt ist und daher bei erneutem Einsatz des Reaktors schnell beheizt werden muss, um die Anfahrtstemperatur zu erreichen.

In einer weiteren vorteilhaften Ausgestaltung und Weiterbildung der Erfindung stammt der Wasserstoff aus der Elektrolyse von Wasser. Besonders vorteilhaft kann der Rohrreaktor mit den drei Reaktionszonen mit dem fluktuierenden Wasserstoffstrom aus der Elektrolyse in Abhängigkeit der zur Verfügung stehenden elektrischen Energie dynamisch betrieben werden.

Weitere Merkmale, Eigenschaften und Vorteile der vorliegenden Erfindung ergeben sich aus der nachfolgenden Beschreibung unter Bezugnahme auf die beiliegenden Figuren. Darin zeigen schematisch:
- Figur 1: einen Rohrreaktor mit drei Reaktionszonen, Reaktionsumsatz und drei Temperaturbereichen;
- Figur 2: eine metallische Trägerstruktur mit Ruthenium umfassender Schicht;
- Figur 3: ein Verfahrensschema der Methanisierung des Wasserstoffs mittels des Rohrreaktors.

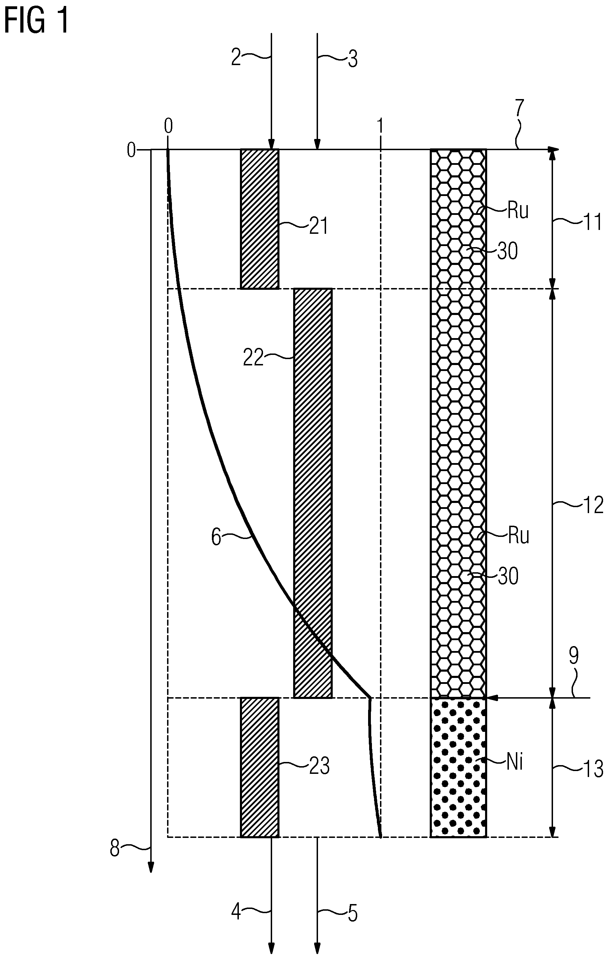

Figur 1 zeigt ein erstes Ausführungsbeispiel des Rohrreaktors 1 mit einer ersten Reaktionszone 11, einer zweiten Reaktionszone 12 und einer dritten Reaktionszone 13. Weiterhin zeigt Figur 1 in Längsrichtung des Rohrreaktors 1, also in Strömungsrichtung des Eduktgases, eine y-Achse 8, welche die Länge des Rohrreaktors 1 beschreibt. Figur 1 zeigt quer zur Strömungsrichtung der Eduktgase eine x-Achse 7, welche sowohl den Reaktionsumsatz als auch die Temperaturbereiche beschreibt.

Die Methanisierungs-Reaktion kann mit Gleichung 1 beschrieben werden.

4 H₂+CO₂→CH₄+2 H₂O Gleichung 1

Figur 1 zeigt, dass die erste Reaktionszone 11 eine metallische Trägerstruktur 30 aufweist. Die metallische Trägerstruktur 30 ist mit einem Ruthenium Ru umfassenden Material beschichtet. Das Ruthenium Ru weist eine höhere katalytische Aktivität für die Methanisierungsreaktion auf als insbesondere Nickel Ni. Diese hohe Aktivität des Rutheniums Ru in Verbindung mit dem Auftragen desselben als Schicht 32 auf eine Struktur 30 mit einer großen Oberfläche ermöglicht ausreichend großen Umsatz in einem, insbesondere im Vergleich zu Nickel basierten Katalysatoren, kompakteren Raum. Vorteilhaft verkleinert dies die Dimensionen des Rohrreaktors 1. Des Weiteren kann diese Zone des Rohrreaktors 1, insbesondere aufgrund der hohen Aktivität des Rutheniums Ru, dynamisch mit unterschiedlicher Menge des Edukts, insbesondere des Wasserstoffs, betrieben werden. Vorteilhaft kann der Rohrreaktor 1, in Abhängigkeit des Verfügbaren Wasserstoffs, dynamisch betrieben werden, wobei komplette Stillstandzeiten vermieden werden.

Die erste Reaktionszone stellt eine Anfahrzone für die Methanisierungs-Reaktion nach Gleichung 1 dar. In dieser ersten Reaktionszone herrscht eine erste Temperatur in einem ersten Temperaturbereich zwischen 100 °C und 400 °C, besonders bevorzugt zwischen 150 °C und 250 °C. Dieser Temperaturbereich ist in Figur 1 als Band dargestellt. Die Temperatur muss zweckmäßigerweise die Reaktionsstart-Temperatur überschreiten. Außerdem sollte die Temperatur oberhalb von 100 °C liegen, damit das während der Reaktion entstehende Wasser dampfförmig vorliegt. Weiterhin zeigt Figur 1 anhand des Umsatzes 6, dass die Reaktion in dieser ersten Reaktionszone 11 startet. Die in der ersten Reaktionszone 11 entstandene Wärmemenge wird verwendet, um die zweite Reaktionszone 12 mittels Konvektion zu beheizen.

Figur 1 zeigt weiterhin, dass der Ruthenium Ru umfassende Katalysator in der zweiten Reaktionszone 12 wiederum als Schicht auf einer metallischen Trägerstruktur 30 vorliegt. Die zweite Reaktionszone 12 weist ein höheres Temperaturniveau als die erste Reaktionszone 11 auf. Die Temperatur in der zweiten Reaktionszone 12 liegt typischerweise maximal bei 600 °C, insbesondere maximal bei 500 °C. Diese maximale Temperatur darf nicht überschritten werden, damit die Festigkeit des Reaktormaterials und die Stabilität des Katalysators, insbesondere des Rutheniums Ru, bestehen bleibt. Aufgrund der hohen Temperaturen und der hohen katalytischen Aktivität des Rutheniums Ru findet in der zweiten Reaktionszone 12 der größte Reaktionsumsatz statt. Allerdings wird der Umsatz in dieser zweiten Reaktionszone 12 durch die Temperatur begrenzt, da die maximale Temperatur trotz der exothermen Reaktion nicht überschritten werden darf.

In einer alternativen, nicht in Figur 1 gezeigten Ausführung, kann das katalytische Material umfassend Ruthenium in der zweiten Reaktionszone auch als Katalysatorbett, insbesondere als Schüttung ausgeführt sein. Vorteilhaft zeigt Ruthenium für die Methanisierungsreaktion insbesondere bei höheren Temperaturen eine bessere Aktivität als Nickel. In der zweiten Reaktionszone ist die Temperatur höher als in der ersten Reaktionszone. Somit kann das Katalysatorvolumen im Vergleich zu ersten Zone geringer sein. Für den Fall, dass der Reaktor etwas weniger flexibel betrieben wird und mit weniger Lastwechsel zu rechnen ist, kann das Ruthenium demnach vorteilhaft als Katalysatorschüttung ausgeführt werden.

Figur 1 zeigt weiterhin, dass in der dritten Reaktionszone 13 der Katalysator Nickel Ni als Katalysatorbett in Form einer Schüttung vorliegt. Die Temperatur in der dritten Reaktionszone 13 liegt unterhalb der Temperatur in der zweiten Reaktionszone 12. Die Temperatur in der dritten Reaktionszone 13 liegt typischerweise in einem Bereich von 200 °C bis 400 °C, insbesondere in einem Bereich von 250 °C bis 350 °C. Um diese Temperaturunterschiede von der zweiten Reaktionszone 12 in die dritte Reaktionszone 13 zu erreichen, bedarf es einer Kühlung entweder zwischen den beiden Reaktionszonen, oder innerhalb der dritten Reaktionszone 13.

Die dritte Reaktionszone dient dazu, den Restanteil an Wasserstoff umzusetzen. Die Wasserstoffmenge, welche die dritte Reaktionszone 13 erreicht, ist nahezu unabhängig von der Eduktmenge an Wasserstoff zu Beginn des Rohrreaktors. Es ist demnach nicht erforderlich, dass die dritte Reaktionszone 13 dynamisch betrieben werden kann. Weiterhin findet in dieser Reaktionszone nicht der größte Umsatz innerhalb des Rohrreaktor statt. Demnach wird hier vorteilhaft Nickel, der im Vergleich zu Ruthenium kostengünstiger ist, aber eine geringere katalytische Aktivität aufweist, als Katalysator eingesetzt. Aufgrund der geringeren Aktivität wird der Nickel umfassende Katalysator insbesondere in Form eine Schüttung in die dritte Reaktionszone eingebracht.

In diesem Ausführungsbeispiel wird mittels der Zuführvorrichtung 9 als Kühlmittel Wasser 5 hinzugegeben. Es handelt sich in diesem Beispiel also um eine direkte Kühlung. Alternativ, aber hier nicht gezeigt, ist es möglich, dass die dritte Reaktionszone 13 mittels Wärmetauscherstrukturen indirekt gekühlt wird. Wasser als Kühlmittel ist insbesondere bei der direkten Kühlung vorteilhaft, da es einerseits, insbesondere aufgrund der Verdampfungsenthalpie, eine ausreichend große Kühlwirkung erzielen kann und andererseits bereits als Produkt im Rohrreaktor 1 vorhanden ist, sodass keine zusätzlichen Komponenten in das System geführt werden müssen.

Wie auch in der ersten Reaktionszone 11 wird der Temperaturbereich nach unten durch die Reaktionsstarttemperatur und die Kondensationstemperatur des Wassers 5 begrenzt. Figur 1 verdeutlicht, dass nach der dritten Reaktionszone 13 nahezu Vollumsatz erreicht wird.

Die erste und die zweite Reaktionszonen 11, 12 ermöglichen durch den Einsatz des Ruthenium Ru umfassenden Katalysators den Betrieb für unterschiedliche Wasserstoff-Eingangskonzentrationen. Aufgrund der für jede Reaktionszone festgelegten Temperaturbänder ist es möglich, den Reaktor dynamisch zu betreiben. Ein großer Wasserstoffspeicher zum Speichern des Wasserstoffs in Zeiten von hoher Wasserstoffproduktion ist vorteilhaft nicht nötig.

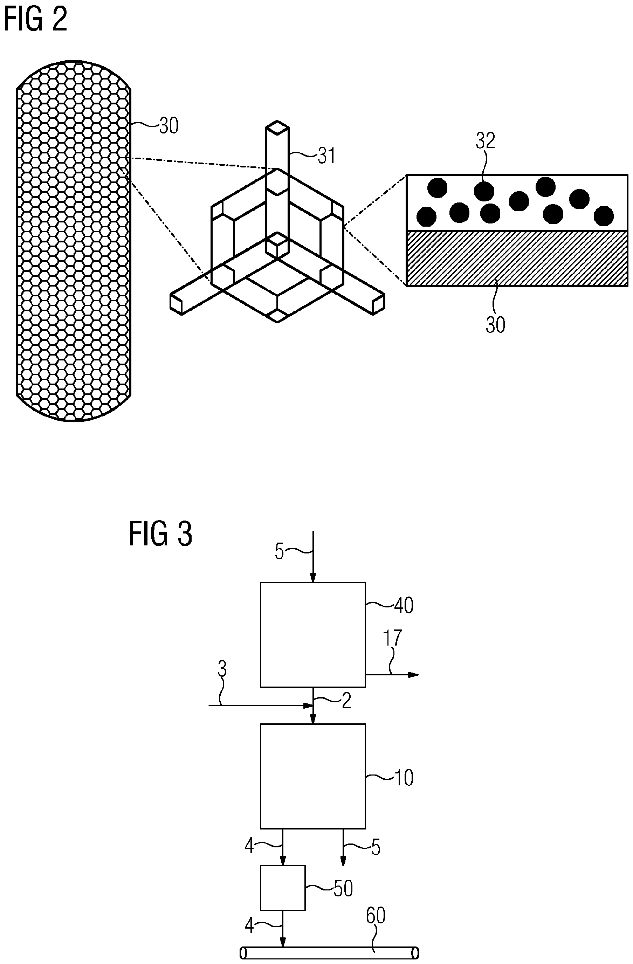

Figur 2 zeigt eine metallische Trägerstruktur 30, auf welche ein Material umfassend Ruthenium Ru als Katalysator aufgebracht wurde. Die metallische Trägerstruktur 30 umfasst somit eine Katalysatorschicht 32. Diese Metallstruktur 30 weist insbesondere die Form einer Diamanteinheitszelle 31 auf, welche Zwischenräume aufweist. Vorteilhaft weist diese metallische Trägerstruktur eine hohe Wärmeleitfähigkeit und eine hohe elektrische Leitfähigkeit auf. Zudem weist diese Struktur ein hohes Oberflächen- zu Volumenverhältnis auf, was einen effektiven Wärmeeintrag in den Reaktor 1 ermöglicht.

Der Wärmeeintrag in den Rohrreaktor 1 kann über die Beheizung einer Reaktorwand des Rohrreaktors 1 erfolgen. Dann ist es zweckmäßig wenn die Reaktorwand des Rohrreaktors 1 die Metallstruktur 30 direkt kontaktiert.

Alternativ ist es besonders bevorzugt, dass die Metallstruktur 30 von Elektroden kontaktiert wird und bei Anlegen einer Spannung aufgrund des elektrischen Widerstands erhitzt wird. Dies ermöglicht eine sehr effiziente und schnelle Erwärmung der Metallstruktur 30, was den Wärmeeintrag in den Rohrreaktor 1 energieeffizient gestaltet. Vorteilhaft kann weiterhin bei der Unterbrechung der Eduktzufuhr der abgekühlte Rohrreaktor 1 schnell elektrisch beheizt werden und somit schnell wieder die Betriebstemperatur der ersten Reaktionszone 11 erzeugt werden. Diese beschichtete Metallstruktur 30 liegt besonders vorteilhaft in der ersten Reaktionszone 11 vor. Es ist aber auch möglich, dass die Metallstruktur 30 auch in der zweiten Reaktionszone 12 und in der dritten Reaktionszone 13 angeordnet ist.

Figur 3 zeigt schematisch die Verschaltung des Rohrreaktors 1, also der Methanisierung 10, mit der Elektrolyse 40, einer Aufbereitungsanlage 50 und dem Erdgasnetz 60.

Wasser 5 wird mittels elektrischer Energie, insbesondere wenn Überschussenergie bei viel Wind und Sonne vorhanden ist, zu Wasserstoff 2 und Sauerstoff 17 gespalten. Der Wasserstoff 2 wird in den Rohrreaktor 1 zu Methanisierung 10 geführt. Außerdem wird Kohlenstoffdioxid 3 zugeführt. Während der Methanisierung 10 wird aus dem Kohlenstoffdioxid 3 und dem Wasserstoff Methan 4 und Wasser 5 hergestellt. Das Methan 4 wird anschließend in eine Methanaufbereitungsanlage 50 geführt, wo es so aufgearbeitet wird, dass es in das Erdgasnetz 60 geleitet werden kann. Alternativ zu dem Führen in die Erdgasleitung ist es denkbar, dass das Methan 4 für Folgereaktionen verwendet wird. Eine weitere Alternative ist das Einsetzen des Methans als Treibstoff, insbesondere für Fortbewegungsmittel, insbesondere Fahrzeuge.

### Bezugszeichenliste

- 1: Rohrreaktor
- 2: Wasserstoff
- 3: Kohlenstoffdioxid
- 4: Methan
- 5: Wasser
- 6: Umsatz
- 7: x-Achse
- 8: y-Achse
- 9: Zuführvorrichtung
- 10: Methanisierung
- 11: erste Reaktionszone
- 12: zweite Reaktionszone
- 13: dritte Reaktionszone
- 14: erstes Katalysatorbett
- 15: zweites Katalysatorbett
- 16: drittes Katalysatorbett
- 17: Sauerstoff
- 21: erster Temperaturbereich
- 22: zweiter Temperaturbereich
- 23: dritter Temperaturbereich
- 30: Metallstruktur
- 31: Diamanteinheitszelle
- 32: Katalysatorschicht
- 40: Elektrolyse
- 50: Methanaufbereitungsanlage
- 60: Erdgasnetz
- Ni: Nickel
- Ru: Ruthenium

## Patentansprüche

1. Rohrreaktor (1) zum Umsetzen von Wasserstoff (2) zu Methan (4) umfassend
- eine erste Reaktionszone (11) mit einer metallischen Trägerstruktur (30), die mit einem Ruthenium (Ru) umfassenden Material (32) beschichtet ist, mit einer Temperatur in einem ersten Temperaturbereich,
- eine zweite Reaktionszone (12) mit einer metallischen Trägerstruktur (30), die mit einer Ruthenium (Ru)umfassenden Material beschichtet ist, oder mit einem als Schüttung ausgeführten Ruthenium (Ru) umfassenden Katalysatorbett mit einer Temperatur in einem zweiten Temperaturbereich, und
- eine dritte Reaktionszone (13) mit einem als Schüttung ausgeführten Katalysatorbett umfassend Nickel (Ni) als Katalysator mit einer Temperatur in einem dritten Temperaturbereich.

2. Rohrreaktor (1) nach Anspruch 1, wobei die metallische Trägerstruktur (3) wabenförmig, porös oder in Form einer Diamanteinheitszelle (31) ausgestaltet ist.

3. Rohrreaktor (1) nach einem der vorhergehenden Ansprüche, wobei die metallische Trägerstruktur des Ruthenium-basierten Katalysators Al₂O₃, Al₂O₃/MgO, CeO₂ oder SiO₂ umfasst.

4. Rohrreaktor (1) nach einem der vorhergehenden Ansprüche, wobei in Strömungsrichtung des Wasserstoffs (2) eingangsseitig die erste Reaktionszone (11), danach die zweite Reaktionszone (12) und danach die dritte Reaktionszone (13) angeordnet ist.

5. Rohrreaktor (1) nach einem der vorhergehenden Ansprüche, wobei der dritte Temperaturbereich unterhalb des zweiten Temperaturbereichs liegt.

6. Rohrreaktor (1) nach einem der vorhergehenden Ansprüche, wobei der zweite Temperaturbereich oberhalb des ersten Temperaturbereichs liegt.

7. Rohrreaktor (1) nach einem der vorhergehenden Ansprüche, wobei der erste Temperaturbereich von 100 °C bis 400 °C reicht.

8. Rohrreaktor (1) nach einem der vorhergehenden Ansprüche, wobei der erste Temperaturbereich von 250°C bis 350°C reicht.

9. Rohrreaktor (1) nach einem der vorhergehenden Ansprüche, wobei der zweite Temperaturbereich maximal bis zu 600 °C reicht.

10. Rohrreaktor (1) nach einem der vorhergehenden Ansprüche, wobei der dritte Temperaturbereich von 200 °C bis 400 °C, insbesondere von 200 °C bis 250 °C, reicht.

11. Rohrreaktor (1) nach einem der vorhergehenden Ansprüche, wobei die erste Reaktionszone (11) eine Heizung umfasst.

12. Rohrreaktor (1) nach einem der vorhergehenden Ansprüche, wobei die poröse Metallstruktur (30) in direktem Kontakt mit der Heizung steht.

13. Verfahren zum Betreiben eines Rohrreaktors (1) mit folgenden Schritten:
- Bereitstellen eines Rohrreaktors (1) mit einer ersten Reaktionszone (11) mit einer metallischen Trägerstruktur (30), die mit einem Ruthenium (Ru) umfassenden Material beschichtet ist, mit einer Temperatur in einem ersten Temperaturbereich,
mit einer zweiten Reaktionszone (12) mit einer metallischen Trägerstruktur (30), die mit einem Ruthenium (Ru) umfassenden Material (32) beschichtet ist, oder mit einem als Schüttung ausgeführten Katalysatorbett mit einem Ruthenium (Ru) umfassenden Material mit einer Temperatur in einem zweiten Temperaturbereich, und mit einer dritten Reaktionszone (13) mit einem als Schüttung ausgeführten Katalysatorbett mit einem Nickel (Ni) umfassenden Material mit einer Temperatur in einem dritten Temperaturbereich,
- Zuführen eines Eduktgases umfassend Wasserstoff (2) in die erste Reaktionszone (11),
- Umsetzen eines ersten Anteils des Wasserstoffs (2) zu einem ersten Anteil Methan (4),
- Führen des nicht reagierten Wasserstoffs (2) und des ersten Anteils Methan (4) in die zweite Reaktionszone (12),
- Umsetzen eines zweiten Anteils des Wasserstoffs (2) zu einem zweiten Anteil Methan (4) in der zweiten Reaktionszone (12), wobei der zweite Anteil Methan (4) größer als der erste Anteil Methan (4) ist,
- Führen des nicht reagierten Wasserstoffs (2), des ersten und des zweiten Anteils an Methan (4) aus der zweiten (12) in die dritte Reaktionszone (13),
- Umsetzen eines dritten Anteils Wasserstoff (4)zu einem dritten Anteil Methan in der dritten Reaktionszone (13).

14. Verfahren nach Anspruch 13, wobei der Wasserstoff (2) mittels Elektrolyse (40) von Wasser (5) hergestellt wird.
